Europäisches Patentamt

European Patent Office

Office européen des brevets

(11) Veröffentlichungsnummer: **0 085 688**
**B1**

(12) # EUROPÄISCHE PATENTSCHRIFT

(45) Veröffentlichungstag der Patentschrift:
**02.04.86**

(21) Anmeldenummer: **82902349.8**

(22) Anmeldetag: **29.07.82**

(86) Internationale Anmeldenummer:
**PCT/DE 82/00156**

(87) Internationale Veröffentlichungsnummer:
**WO 83/00448 (17.02.83** Gazette 83/5)

(51) Int. Cl.⁴: **B 01 J 10/00,** B 01 J 19/12,
C 12 M 1/00, C 12 N 1/12

(54) **VERFAHREN UND ANLAGE ZUM DURCHFÜHREN VON FOTOCHEMISCHEN PROZESSEN.**

(30) Priorität: **30.07.81 DE 3130105**

(43) Veröffentlichungstag der Anmeldung:
**17.08.83 Patentblatt 83/33**

(45) Bekanntmachung des Hinweises auf die Patenterteilung:
**02.04.86 Patentblatt 86/14**

(84) Benannte Vertragsstaaten:
**AT BE CH DE FR GB LI LU NL SE**

(56) Entgegenhaltungen:
**EP - A - 0 010 571**
**DE - A - 2 024 763**
**DE - C - 402 509**
**US - A - 2 732 663**
**US - A - 4 267 038**

(73) Patentinhaber: **SCHICK, Josef-Hubert, Haus Nr. 18,
D-5203 Much-Feld (DE)**

(72) Erfinder: **SCHICK, Josef-Hubert, Haus Nr. 18,
D-5203 Much-Feld (DE)**

(74) Vertreter: **Berkenfeld, Helmut, Dipl.-Ing., An der
Schanz 2, D-5000 Köln 60 (DE)**

ACTORUM AG

## Beschreibung

Die Erfindung betrifft ein Verfahren zum Durchführen von fotochemischen Prozessen, insbesondere zum Züchten von Algen in einer eine Nährlösung enthaltenden Wasserströmung unter Bestrahlung mit Licht, bei dem Flüssigkeit in einer geschlossenen, lichtdurchlässige Wände aufweisenden Kammer im Kreislauf zwischen einem Ein- und Auslass geführt, Gase und Reagenzien zugesetzt und die Kammer einer Lichteinwirkung ausgesetzt wird. Die Erfindung betrifft weiter eine Anlage zum Durchführen dieses Verfahrens mit einer aus einem lichtdurchlässigen Material bestehenden Kammer mit Einläufen für Luft und Wasser und mit Ausläufen. Insbesondere befasst sich die Erfindung jedoch mit einem Verfahren und einer Anlage zum Züchten von Algen. Obwohl sich die Erfindung allgemein bei fotochemischen Prozessen einsetzen lässt, wird sie zur Vereinfachung der Beschreibung im folgenden am Beispiel des Züchtens von Algen beschrieben.

Algen sind primitive Lebewesen, die sich durch einfache Zellteilung fortpflanzen bzw. vermehren. Sie sind Pflanzen. Zu ihrer Vermehrung benötigen sie daher lediglich eine Nährlösung, die vorwiegend anorganische Verbindungen enthält, Kohlendioxyd und Licht. Als Kohlendioxyd reicht der Kohlendioxydanteil der Luft aus. Wegen dieser bescheidenen Anforderungen eignen sich die Algen ausgezeichnet zum Erzeugen sogenannter Biomasse. Dabei kann man die Nährlösung so einstellen, dass die Biomasse vorwiegend Eiweiss bzw. Protein enthält und dann an Vieh verfüttert werden kann oder sehr viel Kohlenstoffverbindungen enthält und dann zur Gewinnung von Alkohol vergoren werden kann.

Das Züchten von Algen in offenen Becken ist bekannt. Dies erfolgt vorwiegend in sogenannten südlichen warmen Ländern, in denen die Algen im Jahresund auch im Tagesdurchschnitt einer starken Sonneneinstrahlung ausgesetzt sind. Die Becken werden mit Wasser und einer Nährlösung gefüllt. Frische Nährlösung wird von Zeit zu Zeit hinzugegeben. Die Becken können weiter kontinuierlich von Wasser durchströmt werden. Die Vorteile einer solchen Züchtung von Algen liegen darin, dass sie wenig maschinellen und personellen Aufwand erfordert. Diese Vorteile werden jedoch mit grossen Nachteilen erkauft. Die Becken sind offen. Deshalb können ständig fremde, andersartige Algen aus benachbarten Gewässern und Feuchtgebieten einfliegen. Die Becken enthalten dann verschiedenartige Algen, die verschiedenartige Wachstumsbedingungen stellen. Deshalb ist es nicht mehr möglich, diese Bedingungen, wie zum Beispiel die Nährlösung, auf optimales Wachstum einzustellen. Ein weiterer Nachteil der Zucht in den offenen Becken liegt darin, dass die grosse Flächen benötigen. Damit das Licht auch bei schon starkem Algenwuchs ausreichend bis zu den nicht unmittelbar an der Oberfläche schwimmenden Algen vordringt, müssen die Becken flach gehalten werden. Deshalb werden grosse Becken benötigt. Dies erhöht wieder die Gefahr, dass andersartige Algen und auch Schmutz eindringen.

Bekannt sind ein Verfahren und eine Anlage (US-A-2 732 663) zum Durchführen von fotochemischen Prozessen, insbesondere zum Züchten von Algen in einer eine Nährlösung enthaltenden Wasserströmung unter Bestrahlung mit Licht. Bei diesem Stand der Technik ist ein aus einem lichtdurchlässigen flexiblen Material bestehendes Rohr in einer horizontalen Ebene hin- und hergehend verlegt. In dem Rohr wird die die Algen enthaltende Nährlösung umgewälzt. Das Rohr schliesst die Nährlösung von der Aussenwelt ab. Schmutz und andersartige Algen können nicht in die Nährlösung eintreten. Trotzdem vermehren sich die Algen nur wenig und die mit diesem bekannten Verfahren erzielbare Ausbeute ist gering. Diese hängt davon ab, in welchem Umfang oder in welcher Anzahl pro Zeiteinheit die Algen mit Luftbläschen und Nährstoffpartikeln in Berührung geraten. Dieser Berührungshäufigkeit ist bei dem bekannten Verfahren eine niedrige obere Grenze gesetzt. Die beiden Enden des hin- und hergehend verlaufenden Rohres sind über eine Leitung miteinander verbunden, die eine Pumpe, Tanks und eine Zuleitung für frische Nährlösung enthält. Das heisst, dass die Strömung auf ihrem langen Weg durch das Rohr im wesentlichen laminar erfolgt. Das Wasser wird kaum durchwirbelt. Nur auf einer geringen Strecke im engeren Einwirkungsbereich der Pumpe tritt eine Turbulenz auf. Nur hier gelangen die Algen in grösserem Umfang mit Luftbläschen und Nährstoffpartikeln in Berührung. Auf dem grössten Teil seines Weges durch das Rohr strömt das Wasser mit den Algen, Nährstoffpartikeln und Luftbläschen ohne Durchwirbelung oder Durchmischung dahin. Hieraus ergibt sich zwangsläufig die geringe Ausbeute des bekannten Verfahrens.

Demgegenüber hat sich der Erfinder die Aufgabe gestellt, das Verfahren mit hoher Ausbeute ablaufen zu lassen. Dieses Ziel wird erfindungsgemäss dadurch erreicht, dass die Flüssigkeit auf einem vertikalen Weg durch eine Kammer durchgeführt, Luft unten in diese eingeleitet, mit der Wasserströmung durchwirbelt und oben aus dieser abgeleitet wird. Das erfindungsgemässe Verfahren verläuft somit in einer vertikalen Ebene. Damit kann die Luft an einer unteren Stelle in den Kreislauf eingeblasen werden. Diese Luft steigt in Form von Blasen mit grosser Oberfläche nach oben. Bei dieser Aufwärtsbewegung bewegen sich die Blasen ständig etwas hin und her. Das heisst, dass die Nährstoffpartikel und die Algen ständig angestossen und hin- und herbewegt werden. Dabei gelangen die Nährstoffpartikel und die Algen ständig mit der grossen Oberfläche der zahlreichen Luftbläschen in Berührung. Damit erfolgt eine innige Durchmischung, die zu einer hohen Ausbeute führt. In der Praxis zeigt sich dies unter anderem darin, dass sich die in einer grossen Kammer enthaltene Nährlösung nach dem Impfen mit einer kleinen Algenmenge schon nach kurzer Zeit wegen des raschen Algenwachstums tiefgrün verfärbt hat.

Das erfindungsgemässe Verfahren kann im Freien und damit unter Sonnenlicht durchgeführt werden. In einer zweckmässigen Ausgestaltung ist jedoch weiter vorgesehen, dass die Kammer mit künstlichem Licht bestrahlt wird.

Das erfindungsgemässe Verfahren läuft in einer geschlossenen, aus einem transparenten Material, zum Beispiel Acrylglas, bestehenden Kammer ab.

0 085 688

Die Kammer ist durch Zwischenwände so unterteilt, dass sich zahlreiche hintereinanderliegende Zellen ergeben, die eine lange Wegstrecke bilden, über die die Algen durch die Kammer durchgeführt werden. Dabei sollte die Kammer so schmal gehalten sein, dass das Licht, Tages- oder künstliches Licht, von beiden Seiten in sie eindringen kann. Damit können sämtliche Algen unabhängig von ihrer Lage in der Kammer an den fotochemischen Reaktionen teilnehmen. Die Kammer wird mit einem Ein- und einem Auslauf an eine in sich geschlossene Wasserströmung angeschlossen. Dieser wird eine definierte Nährlösung zugegeben. Gleichzeitig wird Luft als Träger von Kohlendioxyd zugegeben. Bei den fotochemischen Reaktionen erzeugen die Algen Sauerstoff. Durch Messen des Sauerstoffgehaltes können damit der Stoffwechsel und das Wachstum der Algen gemessen und die Zugabe neuer Nährlösung bestimmt werden. Ebenso lässt sich der pH-Wert auf den für das Wachstum der Algen optimalen Wert einstellen.

Im einzelnen ist erfindungsgemäss vorgesehen, dass die Kammer vertikal angeordnet und durch Zwischenwände in aufeinanderfolgende und einen langen Weg bildende Zellen unterteilt ist, der Einlauf für Luft am unteren Ende der Kammer angeordnet und an eine Luftpumpe angeschlossen ist, der Einlauf für Wasser ebenfalls am unteren Ende der Kammer angeordnet und an den Auslauf der gleichen oder einer in einem Kreislauf vorhergehenden Kammer angeschlossen ist, wobei der Auslauf am oberen Ende einer Kammer angeordnet ist. Dabei hat sich als zweckmässig herausgestellt, dass über dem Boden der Kammer eine poröse Platte angeordnet ist, der Einlauf für Luft unter und der Einlauf für Wasser über der Platte in die Kammer einmündet und in der Kammer zwischen deren Seitenwänden von einer Stelle kurz oberhalb der Platte bis kurz vor deren Oberseite diese in einzelne senkrecht verlaufende Zellen unterteilende, aus einem lichtdurchlässigen Material bestehende Zwischenwände angeordnet sind. Vorteilhaft ist, wenn die Zwischenwände abwechselnd an ihrem unteren und an ihrem oberen Ende Ausnehmungen aufweisen, über die die einzelnen Zellen miteinander verbunden sind. In einer zweckmässigen Ausgestaltung sind die Seiten- und Zwischenwände zu einer Einheit zusammengefasst und diese an ihrem unteren und oberen Ende abgeschlossen. Vorteilhaft ist am Auslass ein Gasabscheider angeordnet, dessen Gasaustritt in die Atmosphäre einmündet und dessen Wasserauslauf zur gleichen oder nächstfolgenden Kammer zurückgeführt ist.

Das erfindungsgemässe Verfahren und die erfindungsgemässe Anlage eignen sich zum Durchführen von Fermentationsprozessen, insbesondere zum Herstellen von mikrobiologischen Produkten, wie Einzellerprotein.

Am Beispiel der in der Zeichnung schematisch gezeigten Ausführungsform einer erfindungsgemässen Anlage wird die Erfindung nun weiter erläutert. In der Zeichnung ist:

Fig. 1 eine schematische Vorderansicht einer erfindungsgemässen Anlage,

Fig. 2 eine Seitenansicht, teilweise im Schnitt, durch eine Kammer der erfindungsgemässen Anlage,

Fig. 3 eine Vorderansicht in stark vergrössertem Massstab auf einige Zellen der erfindungsgemässen Kammer und

Fig. 4 eine gegenüber Fig. 3 um 90° gedrehte Darstellung der die Zellen bildenden Zwischenwände mit den Ausnehmungen am oberen und unteren Ende.

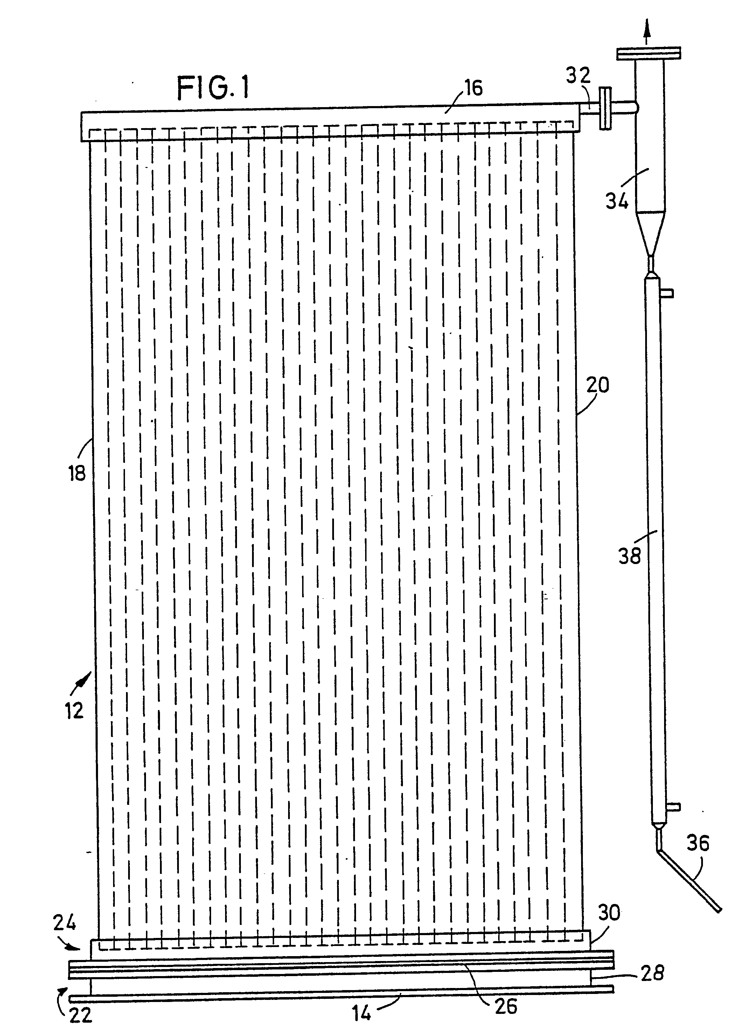

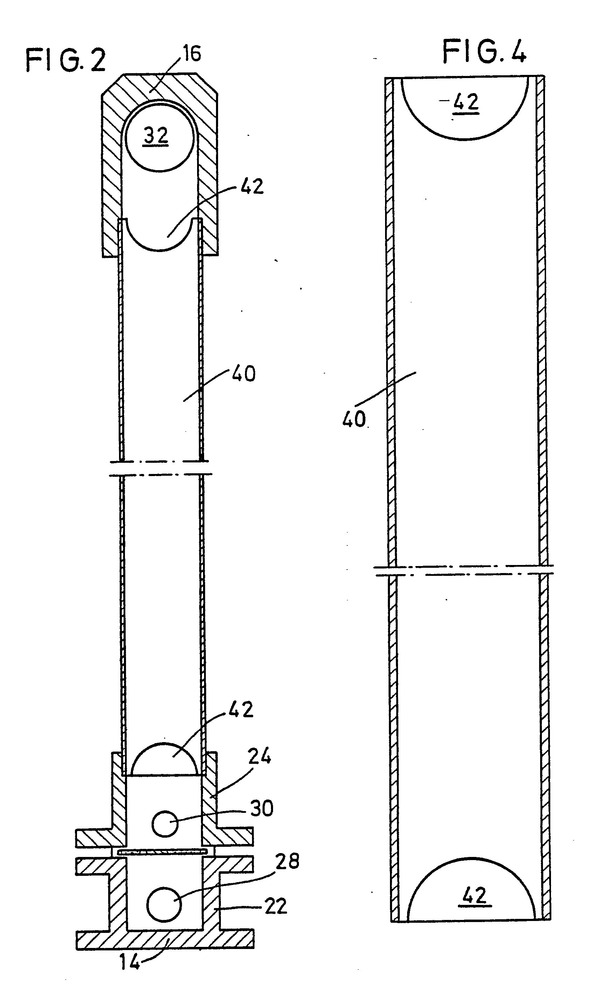

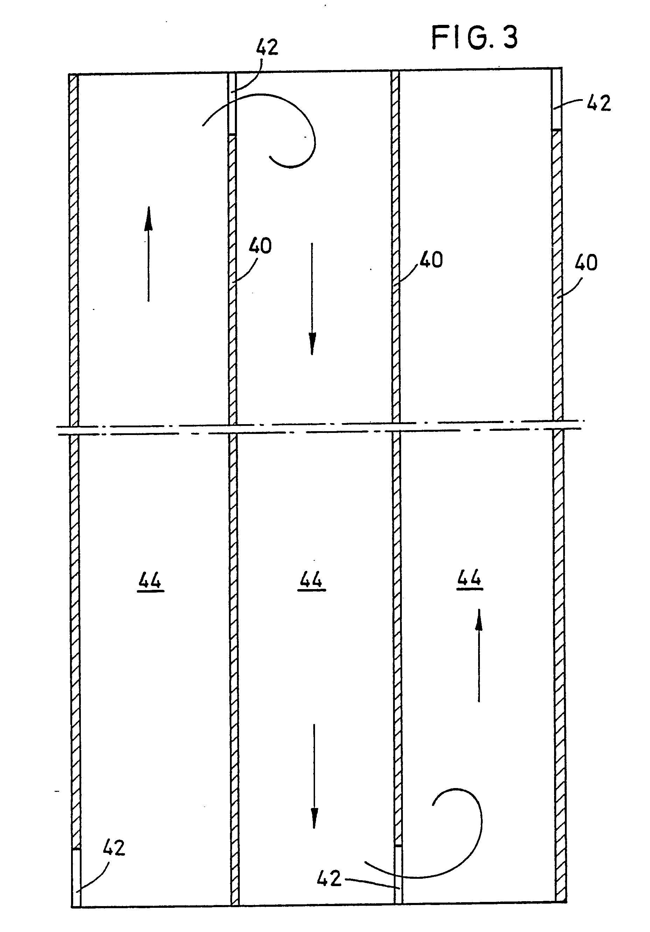

Fig. 1 zeigt eine Kammer 12 mit einem Boden 14, einer Oberseite 16 und zwei Stirnseiten 18 und 20. Wie sich insbesondere aus dem in Fig. 2 gezeigten Schnitt ergibt, besteht die Kammer 12 in ihrem unteren Bereich aus einem unteren kastenförmigen Abschnitt 22 und einem auf diesen aufgesetzten oberen kastenförmigen Abschnitt 24. Zwischen diesen verläuft eine poröse Platte 26. Sie besteht aus Sintermetall oder Kunststoff. Ein Einlauf 28 für Luft mündet in den unteren kastenförmigen Abschnitt 22 und ein Einlauf 30 für Wasser und/oder Nährlösung mündet in den oberen kastenförmigen Abschnitt 24 ein. Am oberen Ende der Kammer 12 ist ein gemeinsamer Auslauf 32 vorgesehen. Gemäss der Darstellung in Fig. 1 mündet dieser in einen Gasabscheider 34. Aus diesem tritt das Gas in Richtung des eingezeichneten Pfeiles nach oben aus, während das Wasser nach unten strömt und in den Ablauf 36 eintritt. Von dort wird es in den Einlauf einer nächsten Kammer eingeleitet oder im Kreislauf in den Einlauf 30 der gleichen Kammer zurückgeführt. Hierauf wird noch eingegangen werden. Auf seinem Weg vom Gasabscheider 34 zum Ablauf 36 durchläuft das Wasser einen Kühlmantel 38. Dieser ist über die gezeigten Rohrstutzen an einen Kühlmittelkreislauf angeschlossen. Die Kammer 12 wird durch einzelne Zwischenwände 40, die abwechselnd an ihren unteren und oberen Enden Ausnehmungen 42 aufweisen, in Zellen 44 unterteilt. Fig. 3 zeigt vier solche Zwischenwände 40 mit den zwischen ihnen gebildeten Zellen 44. Zur Vereinfachung der Darstellung sind die Zwischenwände 40 in der Mitte gebrochen. Fig. 3 zeigt nur ihre unteren und oberen Enden. Fig. 3 zeigt weiter den Strömungsverlauf durch die benachbarten Zellen 44. Die Strömung erfolgt in einer Zelle 44 nach oben. Durch eine obenliegende Ausnehmung 42 geht sie in die benachbarte Zelle 44 über und verläuft in dieser nach unten. Aus dieser tritt sie durch eine untenliegende Ausnehmung 42 in die nächste Zelle 44 ein und verläuft in dieser wieder nach oben. Diese Strömungsrichtungen werden durch die Pfeile angezeigt. Die in die Ausnehmungen 42 eingezeichneten bogenförmigen Linien bedeuten, dass hier Wirbel entstehen. Die Darstellung in Fig. 4 erläutert weiter, dass die Ausnehmungen 42 abwechselnd am unteren und oberen Ende angeordnet sind.

Sämtliche Wände der Kammer 12 einschliesslich der Zwischenwände bestehen aus einem transparenten Material, wie Acrylglas oder zum Beispiel Plexiglas (eingetragenes Warenzeichen). Der Einlauf 28 der Kammer ist an eine Luftpumpe angeschlossen. Falls die Kammer im Kreislauf arbeitet, ist ihr Einlauf 30 an ihren eigenen Ablauf 36 angeschlossen. Falls mehrere Kammern, bis etwa zehn, hintereinander oder in Serie geschaltet sind, ist der Einlauf 30 einer Kammer an den Ablauf 36 der jeweils vorhergehenden Kammer angeschlossen. Zu Beginn eines Arbeitsablaufes werden dem Wasser eine geringe Menge der ausgewählten Algenart und die Nährlösung zugesetzt. Die durch den Einlauf 28 eingeblasene Luft trifft auf die poröse Platte 26 und verlässt diese in Form von sehr feinen Blasen. Diese haben eine Grösse von vorzugsweise 1 bis 5 µ. Zu Beginn eines Arbeitsablaufes steigen diese Luftbläschen in sämtlichen Zellen 44 gleichmässig nach oben. Nach einer kurzen Zeit ergibt sich jedoch der in Fig. 3 durch die Pfeile angezeigte Kreislauf. In einer Zelle perlen die Luftbläschen nach oben, während sie in der nächstbenachbarten Zelle nach unten strömen. Dabei reicht der Auftrieb, der durch die in jeder zweiten Zelle nach oben perlenden Luftblasen bewirkt wird, aus, um das Wasser in diesen Zellen nach oben zu tragen und in den jeweils benachbarten Zellen nach unten zu drücken. Es entsteht ein «air lift». Die durch die Luftpumpe eingeführte kinetische Energie reicht aus, um das Wasser in der oder den Kammern in Umlauf zu halten. Besondere Wasserpumpen sind nicht erforderlich. Die in das Wasser eingegebenen Algen und die Nährlösung durchströmen die Zellen gemeinsam in der gleichen Richtung. Bei ihrem Durchtritt durch die Zellen 44 kommen die Algen mit den feinen Luftbläschen in Berührung. Wegen der feinen Aufteilung der Luft in kleine Bläschen ergeben sich wegen der grossen Bläschenoberfläche pro m² Zellenfläche 50 m² Luftoberfläche. Damit kommen die Algen auf einer grossen Fläche mit Kohlendioxyd in Berührung. Gleichzeitig werden sie von sämtlichen Seiten Licht ausgesetzt. Durch die hinter und vor der Zeichenebene liegenden Seitenwände der Kammer 12 tritt das Licht auch in die Zwischenwände 40 ein. Damit erhalten die in den Zellen 44 befindlichen Algen von sämtlichen vier Seiten Licht. Die Zellen 44 haben ein Innenmass von etwa 8 bis 40 mm, vorzugsweise 40 mm. Bei erfindungsgemäss erreichbaren optimalen Bedingungen können die Algen dann ihr Gewicht in etwa vier Stunden verdoppeln. Hierbei durchlaufen sie, wie die Figuren 1 und 3 zeigen, eine lange, in etwa zickzackförmige Bahn, auf der die Wasserströmung mit den Luftbläschen und der Nährlösung durch die Kammer 12 durchbewegt wird. Im Gasabscheider 34 findet eine Entgasung statt. Eine optimal grosse Berührungsfläche zwischen Algen und Luft und eine maximale Strömungsgeschwindigkeit werden damit sichergestellt. Falls die Temperatur der Wasserströmung durch die Einwirkung des Lichtes, wobei es sich vorzugsweise um Sonnenlicht handelt, über einen für das Wachstum der Algen optimalen Wert angestiegen sein sollte, wird der Kühlmittelkreislauf zum Kühlmantel 38 angeschaltet und die Wasserströmung gekühlt.

Die Anwendung der Erfindung ist unabhängig von der Algenart. Besonders gutes Wachstum zeigen jedoch Grünalgen, wie Chlorella fusca und Chlorella vulgaris.

Als Nährlösung haben sich gemäss der Erfindung folgenden Zusammensetzungen als besonders vorteilhaft herausgestellt:

a) 0,5 g Monoammonphosphat
   0,5 g Harnstoff oder Ammoniumnitrat als Stickstoffquelle
   0,18 g CaCl₂

0,1 g KCl
0,25 g MgSO₄·7H₂O
5 ml 0,1 N FeCl₃·6H₂O + Komplexon III
5 ml Spurenlösung
ad 1000 ml Wasser

b) 1 g Monoammonphosphat
   0,1 g KCl
   0,15 g CaCl₂
   0,25 g MgSO₄·7H₂O
   5 ml 0,1 N FeCl₃·6H₂O + Komplexon III

c) 1,2 g Harnstoff oder Ammoniumnitrat
   0,1 g KCl
   0,15 g CaCl₂
   0,25 g MgSO₄·7H₂O
   5 ml 0,1 N FeCl₃·6H₂O + Komplexon III
   5 ml Spurenlösung

d) 7,8 g Harnstoff
   2 g MgSO₄·7H₂O
   3,9 g K₂HPO₄
   10 ml Spurenlösung
   5 ml Fe-Komplex.

Statt der 7,8 g Harnstoff können auch 10 g Ammonnitrat NH₄NO₃ verwendet werden.

Als Spurenlösung hat sich folgende Zusammensetzung erfindungsgemäss als vorteilhaft herausgestellt:

Spurenlösung (pro Liter):
3,1 g H₃BO₃
1,69 g MnSO₄·4H₂O
88 mg (NH₄)₆Mo₇O₂₄·4H₂O
83 mg KJ
0,3 g ZnSO₄·7H₂O
0,125 g Cu(SO₄)·5H₂O.

Zum optimalen Wachstum muss der pH-Wert auf einen bestimmten Bereich eingestellt werden. Hier empfiehlt sich gemäss der Erfindung, den pH-Wert mit Salzsäure HCl auf einen Wert von 6 bis 7, vorzugsweise 6,8, zu bringen.

Wie ausgeführt, werden dem Wasser zu Beginn eines Arbeitsablaufes eine geringe Menge der ausgewählten Algenart und die Nährlösung zugesetzt. Während eines Arbeitsablaufes, dessen Dauer insbesondere von der Art und Stärke der Lichteinstrahlung abhängt, werden die Konzentration der Nährlösung und der pH-Wert möglichst konstant gehalten und hierzu von Zeit zu Zeit Nährlösung zugesetzt. Bei Kunstlicht ist die Lichteinstrahlung konstant. Bei Sonnenlicht hängt sie vom Wetter, der Wolkenbildung usw. ab. Es wurde ausgeführt, dass eine einzige Kammer und auch mehrere Kammern hintereinander in Serie in einem geschlossenen Kreislauf betrieben werden können. Dabei lassen sich zehn oder noch mehr Kammern hintereinanderschalten. Bei Betreiben einer einzigen Kammer wird der Kreislauf unterbrochen, sobald sich die Algen durch Teilung bis auf die gewünschte Masse vermehrt haben und die gewünschte Algenkonzentration erreicht ist. Die Algen werden abgefiltert und einer Nutzung zugeführt. Anschliessend wird die Anlage erneut in Betrieb gesetzt. Bei Hintereinanderschaltung von mehreren Kammern kann dagegen kontinuierlich gearbeitet werden. An der ersten und gegebenenfalls auch an weiteren Kammern wird Nährlösung zugegeben. An der letzten Kammer läuft das Algenkonzentrat ab und wird seiner Nutzung zugeführt. Wasserverluste werden ersetzt. Durch Hintereinanderschaltung von mehreren Kammern lässt sich damit ein kontinuierlicher Verfahrensablauf erreichen. Für eine grosstechnische Anwendung ist dies von besonderer Bedeutung.

Wie ausgeführt, werden die Durchströmung und der Kreislauf allein mit der eingeleiteten Luft bewirkt. Die Luft, die in erster Linie zur Zufuhr von $CO_2$ zu den Algen dient, hat normalerweise einen $CO_2$-Gehalt von 0,03%. Durch Erhöhen des $CO_2$-Gehaltes auf 0,3% lässt sich der Algenertrag auf den zwei- bis vierfachen Wert steigern.

Wie ausgeführt, haben die Zellen 44 vorzugsweise ein Innenmass von 40 mm. Ein vorteilhaftes Mass für die Breite einer Kammer 12 liegt bei 1,20 m. Die Höhe einer Kammer 12 kann zwischen 1,60 und 6,00 m liegen. Der Abstand zwischen der porösen Platte 26 und den Unterkanten der Zwischenwände 40 — siehe Fig. 2 — beträgt vorzugsweise 60 mm. Der Abstand zwischen den Oberkanten der Zwischenwände 40 und der Oberkante des Kanals 32 beträgt ebenfalls vorzugsweise 60 mm.

Die Menge der in jede Kammer pro Zeiteinheit eingeblasenen Luft hängt von vielen Faktoren, unter anderem der Grösse der Kammer ab. Als Anhalt sei gesagt, dass pro Kammer 80 bis 10 l/min Luft eingeblasen werden.

Die erfindungsgemässe Anlage lässt sich auch zum Durchführen von biologischen Fermentationsprozessen verwenden. Sie eignet sich insbesondere zum Herstellen von mikrobiologischen Produkten wie Einzellerproteinen. Bei diesen Herstellungsverfahren kommt es darauf an, Nährlösung und Impfmaterial oder ein Kulturmedium zusammenzubringen, auf einem grösseren Weg oder während einer längeren Zeit miteinander reagieren zu lassen, an einem Punkt dieses Weges Sauerstoff fein verteilt zuzuführen und bei der Fermentation entstehende Wärme abzuleiten. Die erfindungsgemässe Anlage erlaubt dieses Zusammenführen und Reagieren der genannten Stoffe und die Ableitung der exothermen Wärme. Abhängig von der gewünschten Ausbeute und der zur Verfügung stehenden Zeit wird eine Kammer in sich kurzgeschlossen oder mehrere Kammern werden hintereinandergeschaltet. Damit lässt sich die erfindungsgemässe Anlage vorteilhaft anstelle der in der deutschen Patentschrift 2 700 697 beschriebenen Vorrichtung verwenden. Es ist nicht nötig, dass die Kammerwände und -zwischenwände aus transparentem Material bestehen. Gegenüber der in der deutschen Patentschrift beschriebenen Vorrichtung zeichnet sich die erfindungsgemässe Anlage durch geringe Herstellungskosten aus.

**Patentansprüche**

1. Verfahren zum Durchführen von fotochemischen Prozessen, insbesondere zum Züchten von Algen in einer eine Nährlösung enthaltenden Wasserströmung unter Bestrahlung mit Licht, bei dem Flüssigkeit in einer geschlossenen, lichtdurchlässige Wände aufweisenden Kammer im Kreislauf zwischen einem Ein- und Auslass geführt, Gase und Reagenzien zugesetzt und die Kammer einer Lichteinwirkung ausgesetzt wird, dadurch gekennzeichnet, dass die Flüssigkeit auf vertikalem Weg durch die Kammer durchgeführt, Luft unten in die Kammer eingeleitet, mit der Wasserströmung durchwirbelt und oben aus der Kammer abgeleitet wird.

2. Verfahren nach Anspruch 1, dadurch gekennzeichnet, dass die Kammer mit künstlichem Licht bestrahlt wird.

3. Anlage zum Durchführen des Verfahrens nach den Ansprüchen 1 und 2 mit einer aus einem lichtdurchlässigen Material bestehenden Kammer (12) mit Einläufen (28, 30) für Luft und Wasser und Ausläufen (36), dadurch gekennzeichnet, dass die Kammer (12) vertikal angeordnet und durch Zwischenwände (40) in aufeinanderfolgende und einen langen Weg bildende Zellen (44) unterteilt ist, der Einlauf (28) für Luft am unteren Ende der Kammer (12) angeordnet und an eine Luftpumpe angeschlossen ist, der Einlauf (30) für Wasser ebenfalls am unteren Ende der Kammer (12) angeordnet und an den Auslauf (36) der gleichen oder einer in einem Kreislauf vorhergehenden Kammer (12) angeschlossen ist, wobei der Auslauf (36) am oberen Ende einer Kammer (12) angeordnet ist.

4. Anlage nach Anspruch 3, dadurch gekennzeichnet, dass über dem Boden (14) der Kammer (12) eine poröse Platte (16) angeordnet ist, der Einlauf (28) für Luft unter und der Einlauf (30) für Wasser über der Platte (26) in die Kammer (12) einmündet und in der Kammer (12) zwischen deren Seitenwänden von einer Stelle kurz oberhalb der Platte (26) bis kurz vor deren Oberseite diese in einzelne senkrecht verlaufende Zellen (44) unterteilende, aus einem lichtdurchlässigen Material bestehende Zwischenwände (40) angeordnet sind.

5. Anlage nach Anspruch 3 und 4, dadurch gekennzeichnet, dass die Zwischenwände (40) abwechselnd an ihrem unteren und an ihrem oberen Ende Ausnehmungen (42) aufweisen, über die die einzelnen Zellen (44) miteinander verbunden sind.

6. Anlage nach Anspruch 3 bis 5, dadurch gekennzeichnet, dass die Seiten- und Zwischenwände (40) zu einer Einheit zusammengefasst sind und diese an ihrem unteren und oberen Ende abgeschlossen ist.

7. Anlage nach Anspruch 3 bis 6, dadurch gekennzeichnet, dass am Auslauf (36) ein Gasabscheider (34) angeordnet ist, dessen Gasaustritt in die Atmosphäre einmündet und dessen Wasserauslauf zur gleichen oder nächstfolgenden Kammer zurückgeführt ist.

8. Verwendung der Anlage nach den Ansprüchen 3 bis 7 zum Durchführen von Fermentationsprozessen, insbesondere zum Herstellen von mikrobiologischen Produkten, wie Einzellerprotein.

**Claims**

1. A method of carrying out photochemical processes, in particular for growing algae in a flow of water containing a nutrient solution, with irradiation with light, wherein liquid is circulated between an inlet and an outlet in a closed chamber having translucent walls, gases and reagents are added and the chamber is exposed to the effect of light, characterised in that the liquid is passed vertically through the chamber, and air is introduced into the chamber at the bottom, mixed turbulently with the flow of water and removed from the chamber at the top.

2. A method according to claim 1 characterised in that the chamber is irradiated with artificial light.

3. Apparatus for carrying out the method according to claims 1 and 2 including a chamber (12) comprising a translucent material, with inlets (28, 30) for air and water and outlets (36), characterised in that the chamber (12) is disposed vertically and is subdivided by partitions (40) into successive cells (44) forming a long path, the inlet (28) for air is arranged at the lower end of the chamber (12) and is connected to an air pump, the inlet (30) for water is also arranged at the lower end of the chamber (12) and is connected to the outlet (36) of the same chamber (12) or a preceding chamber (12) in a circuit, the outlet (36) being disposed at the upper end of a chamber (12).

4. Apparatus according to claim 3 characterised in that a porous plate (26) is disposed above the bottom (14) of the chamber (12), the inlet (28) for air opens into the chamber (12) below the plate (26) and the inlet (30) for water opens into the chamber (12) above the plate (26) and disposed in the chamber (12) between the side walls thereof, from a location just above the plate (26) to just before the top side thereof, are partitions (40) which subdivide the chamber into individual, vertically extending cells (44) and which comprise a translucent material.

5. Apparatus according to claim 3 and claim 4 characterised in that the partitions (40) are alternately provided at their lower and upper ends openings (42) by way of which the individual cells (44) are in communication with each other.

6. Apparatus according to claims 3 to 5 characterised in that the side walls and partitions (40) are combined together to form a unit and same is closed off at its upper and lower ends.

7. Apparatus according to claims 3 to 6 characterised in that disposed at the outlet (36) is a gas separator (34), the gas outlet of which opens to atmosphere and the water outlet of which is taken back to the same or the next following chamber.

8. Use of the apparatus according to claims 3 to 7 for carrying out fermentation processes, in particular for producing microbiological products such as single-cell protein.

**Revendications**

1. Procédé pour la conduite de processus photochimiques, en particulier pour la culture d'algues dans un courant d'eau contenant une solution nutritive sous irradiation au moyen de lumière, dans lequel le liquide est conduit en circuit entre une entrée et une sortie dans une chambre fermée comportant des parois perméables à la lumière, des gaz et des réactants sont ajoutés et la chambre est exposée à une action de la lumière, caractérisé en ce que le liquide est conduit en trajet vertical à travers la chambre, de l'air est admis par le dessous dans la chambre, dispersé par bouillonnement dans le courant d'eau et évacué par le dessus hors de la chambre.

2. Procédé suivant la revendication 1, caractérisé en ce que la chambre est irradiée au moyen de lumière artificielle.

3. Installation pour la conduite du procédé suivant les revendications 1 et 2, comprenant une chambre (12) constituée d'une matière perméable à la lumière qui comporte des admissions (28, 30) pour l'air et l'eau et une sortie (36), caractérisée en ce que la chambre (12) est agencée verticalement et est divisée par des cloisons (40) en cellules (44) successives constituant un long trajet, l'admission (28) pour l'air est agencée à l'extrémité inférieure de la chambre (12) et est raccordée à une pompe à air, l'admission (30) pour l'eau est agencée aussi à l'extrémité inférieure de la chambre (12) et est raccordée à la sortie (36) de la même chambre ou d'une chambre (12) qui la précède dans un circuit, la sortie (36) étant agencée à l'extrémité supérieure d'une chambre (12).

4. Installation suivant la revendication 3, caractérisée en ce qu'une plaque poreuse (26) est agencée au-dessus du fond (14) de la chambre (12), l'admission (28) pour l'air débouche au-dessous et l'admission (30) pour l'eau au-dessus de la plaque (26) dans la chambre (12) et des cloisons (40) consistant en une matière perméable à la lumière sont agencées dans la chambre (12) entre ses parois latérales depuis un endroit peu au-dessus de la plaque (26) jusqu'à peu au-dessous de sa face supérieure et la divisent en cellules distinctes (44) disposées verticalement.

5. Installation suivant les revendications 3 et 4, caractérisée en ce que les cloisons (40) comportent alternativement à leur extrémité inférieure et à leur extrémité supérieure, des évidements (42) par l'intermédiaire desquels les cellules distinctes (44) communiquent entre elles.

6. Installation suivant les revendications 3 à 5, caractérisée en ce que les parois latérales et cloisons (40) sont assemblées en une unité et celle-ci est fermée à leurs extrémités supérieures et inférieures.

7. Installation suivant les revendications 3 à 6, caractérisée en ce qu'il est agencé à la sortie (36), un séparateur de gaz (34) dont la sortie de gaz débouche à l'atmosphère et dont la sortie d'eau est ramenée à la même chambre ou à une chambre suivante.

8. Utilisation de l'installation suivant les revendications 3 à 7 pour la conduite de processus de fermentation, en particulier pour la fabrication de produits microbiologiques tels que des protéines d'unicellulaires.

FIG.1

FIG. 2

FIG. 4

FIG. 3